Europäisches Patentamt

European Patent Office

Office européen des brevets

(19)

(11) Numéro de publication : **0 380 402 B1**

(12) **FASCICULE DE BREVET EUROPEEN**

(45) Date de publication du fascicule du brevet :
20.05.92 Bulletin 92/21

(21) Numéro de dépôt : **90400177.3**

(22) Date de dépôt : **23.01.90**

(51) Int. Cl.$^5$ : **C07D 493/04,** C07D 307/20

(54) **Procédé de production d'anhydrosorbitols et d'anhydromannitols à partir de sorbitol et de mannitol.**

(30) Priorité : **26.01.89 FR 8901096**

(43) Date de publication de la demande :
**01.08.90 Bulletin 90/31**

(45) Mention de la délivrance du brevet :
**20.05.92 Bulletin 92/21**

(84) Etats contractants désignés :
**AT BE CH DE DK ES FR GB GR IT LI LU NL SE**

(56) Documents cités :
**GB-A- 600 870**
**US-A- 4 313 884**

(73) Titulaire : **INSTITUT FRANCAIS DU PETROLE**
**4, Avenue de Bois Préau**
**F-92502 Rueil-Malmaison (FR)**
Titulaire : **Groupement d'Intérêt Economique**
**dit: SUCRE RECHERCHES ET**
**DEVELOPPEMENTS**
**27-29, Rue Chateaubriand**
**F-75383 Paris Cédex 08 (FR)**

(72) Inventeur : **Barbier, Jacques**
**Les Fousserettes**
**F-86000 Montamise (FR)**
Inventeur : **Boitiaux, Jean-Paul**
**4, Avenue des Ursulines**
**F-78300 Poissy (FR)**
Inventeur : **Chaumette, Patrick**
**34, Côte de la Jonchère**
**F-78380 Bougival (FR)**
Inventeur : **Leporq, Serge**
**6, Rue Villandry**
**F-78200 Mantes-La-Ville (FR)**
Inventeur : **Menezo, Jean-Christophe**
**31, Rue de Malherbe**
**F-86000 Poitiers (FR)**
Inventeur : **Montassier, Claude**
**15, Chiron des Trois Fusées - Sèvres**
**Anxaumont**
**F-86800 Saint-Julien L'Ars (FR)**

(74) Mandataire : **Andreeff, François**
**INSTITUT FRANCAIS DU PETROLE 4, avenue**
**de Bois-Préau**
**F-92502 Rueil-Malmaison (FR)**

## Description

L'invention a pour objet un procédé de production de composés anhydrosorbitols et/ou mannitols à partir de sorbitol et/ou de mannitol sur des catalyseurs multimétalliques à base de cuivre.

L'utilisation des sucres du type glucose, fructose, sorbitol et mannitol en alimentation est pratiquée depuis de nombreuses années car ces sucres ont des propriétés intéressantes qui, dans certains cas, les font préférer au sucrose habituel (saccharose). On peut par exemple citer les propriétés dessicantes du sorbitol qui le rendent intéressant pour certaines formulations pharmaceutiques, ou son intérêt pour les régimes diététiques, car il est peu assimilé par l'organisme humain.

D'autres utilisations plus chimiques sont envisageables et l'on peut citer notamment les produits de cyclisation du type isosorbides et isomannides.

Leur préparation est généralement réalisée en traitant le sorbitol ou le mannitol par un acide minéral comme l'acide sulfurique (brevet DE 3521809 ; G. Flèche et al. Starch/Stärke 38 (1986), 1, 26 ; K. Bock et al. Acta Chemica Scandinavia B 35 (1981), 441), l'acide acétique (brevet DE 31 11092) ou encore en présence d'une résine échangeuse fortement acide (brevet DE 30 41673, brevet japonais 55/79382). Ce mode de préparation donne des rendements initiaux satisfaisants en monoanhydro et dianhydrosorbitol ou mannitol mais une quantité importante de produits polymérisés rend le procédé difficile à mettre en oeuvre.

L'utilisation de catalyseurs métalliques sous pression d'hydrogène a été proposée pour hydrogénolyser le sorbitol et le mannitol en polyols non cycliques du type glycérol, propanediol, butanediol.... Ces catalyseurs sont du nickel sur kieselguhr, du chromate de cuivre, du cobalt sur silice-alumine (brevet US 3,471,580 et US 4,380,678), du ruthénium sulfuré (brevet US 4,476,331) ou du cuivre de Raney (brevet FR 2 603 276).

L'objet de l'invention est d'utiliser un catalyseur métallique permettant d'obtenir à partir de sorbitol et/ou de mannitol des composés anhydrosorbitols et/ou anhydromannitols au lieu d'obtenir les polyols linéaires habituels, tout en évitant les problèmes de polymérisation que présentent les synthèses basées sur l'utilisation d'acides minéraux et de résines échangeuses acides. Le produit de départ peut également être constitué de glucose et/ou fructose et de leurs mélanges. On les soumettra alors, au préalable, à une hydrogénation.

Il a en effet été constaté que, de manière surprenante, la modification d'un catalyseur au cuivre par au moins un métal du groupe VIII, par exemple le palladium, le platine, le ruthénium ou par l'or, change totalement le caractère des transformations que le catalyseur obtenu peut promouvoir. Alors que le cuivre permet l'obtention d'un mélange de polyols linéaires qui sont le butanetriol, le butanediol, le glycérol, le propanediol et les monoalcools correspondants, le solide contenant du cuivre associé à au moins un métal de la liste ci-dessus catalyse une réaction de cyclisation du sorbitol, du mannitol ou de leur mélange, produisant majoritairement un mélange d'anhydrosorbitol (en particulier de 1,4 - anhydrosorbitol) et de dianhydrosorbitol (en particulier le 1,4-3,6 dianhydrosorbitol) et/ou d'anhydromannitol (en particulier le 1,4 anhydromannitol) et de dianhydromannitol (en particulier le 1,4-3,6 dianhydromannitol).

Le catalyseur peut être sous forme de poudre, tel qu'on l'obtient par exemple en imprégnant un charbon actif ou tel qu'on l'obtient en attaquant à la soude un alliage de cuivre Raney et on pourra l'utiliser après modification par au moins un métal du groupe VIII et/ou par l'or.

Le catalyseur peut être également utilisé en lit fixe et sera dans ce cas supporté sur tout support approprié comme le kieselguhr, l'alumine, la silice ou le charbon granulé ; il pourra le terme de coprécipitation ou de comalaxage qui permettent d'obtenir des catalyseurs massiques et ensuite mis en forme au moyen de tout procédé connu tel que l'extrusion, la dragéification, le pastillage ou la coagulation en gouttes, tel que décrit dans les brevets US 4,257,920 et US 4,126,581.

L'adjonction d'un métal du groupe VIII et/ou de l'or peut être réalisée selon le cas par l'une des techniques appropriées dont on donne ci-dessous quelques exemples. On peut citer les techniques de coimprégnation dans le cas des catalyseurs supportés, les techniques de coprécipitations dans le cas des catalyseurs massiques, les techniques par imprégnations successives dans tous les cas : poudre de Raney, catalyseurs supportés ou catalyseurs massiques.

Les conditions dans lesquelles s'effectue la réaction catalytique pourront être par exemple de $10^5$ - $10^7$ Pa de pression d'hydrogène et de préférence de $10^6$ à $5.10^6$ Pa entre 200 et 280°C et de préférence entre 220 et 260°C.

Dans le cas de l'utilisation d'un procédé avec catalyseur sous forme de poudre, on séparea les produits de la réaction du catalyseur par tout moyen connu de l'homme de l'art, par exemple grâce à une filtration ; les composés anhydrosorbitols et anhydromannitols peuvent être ensuite concentrés par distillation de l'eau formée puis pourront par exemple être obtenus purs par cristallisation comme l'enseigne le brevet US 4,564,692.

Les catalyseurs considérés contiennent avantageusement de 10 à 99 % en poids de cuivre et de 0,01 à 10 % en poids d'au moins un second métal de la liste ci-dessus. Le total pourra être égal à 100 % ou l'emploi d'un support pourra compléter le chiffre à 100, s'il lui est inférieur.

On rapportera de préférence la quantité de métal ajouté à la teneur en cuivre de surface. Cette mesure de la quantité de cuivre en surface est réalisée par un titrage des atomes de cuivre de surface par $N_2O$ (protoxyde d'azote) selon la réaction :

$$N_2O + 2Cu \rightarrow N_2 + Cu_2O$$

On pourra utilement consulter la référence suivante pour plus de détails sur cette mesure :

B. Dvorak et J. Pasek J. Catal.18,108 (1970)

La teneur en second métal ajouté au cuivre sera dans ce cas de préférence comprise entre 0,1 et 0,8 atome-gramme de métal ajouté par atome-gramme de cuivre en surface du précurseur de catalyseur. On entend ici par le terme précurseur, le catalyseur au cuivre avant l'introduction du métal modificateur.

Les composés du cuivre utilisables pour la préparation des catalyseurs sont, de façon non limitative : l'alliage aluminium-cuivre, les sels tels que acétate, nitrate, ammine nitrate, carbonates, l'acétylacétonate ou l'octoate.

Les composés des métaux du groupe VIII et de l'or pourront être par exemple des chlorures, des amines chlorures, des nitrates, des carbonyles.

Exemple 1 :

On prépare divers catalyseurs selon l'invention de la façon suivante :

1°) on dépose 20 % poids de cuivre sur du charbon en poudre d'une surface de 1100 m2/g à partir de nitrate de cuivre en solution dans l'eau, que l'on précipite par ajout de soude. Après lavage à l'eau et réduction à 250°C sous courant d'azote renfermant 3 % en volume d'hydrogène, le dosage du cuivre de surface par $N_2O$ indiquant 0,30 $10^{-3}$ atome-gramme de cuivre par gramme de catalyseur, on dépose 0,10 $10^{-3}$ atome-gramme de ruthénium par gramme de catalyseur par échange pendant 4 heures avec une solution de chlorure de ruthénium.

Le catalyseur préparé, une fois séché, constitue le catalyseur A des exemples suivants .

2°) A partir du même précurseur de cuivre sur charbon, on dépose 0,20 $10^{-3}$ atome-gramme de platine par échange pendant 4 heures avec de l'acide chloroplatinique. Le solide obtenu, une fois séché, consitue le catalyseur B.

3°) On dépose 10 % de cuivre sur une silice de 300 m2/g, se présentant sous forme de billes de 2 mm, de la façon suivante : on traite le support à l'ammoniaque à un pH de 10 pendant 1 heure, puis on remplace la solution ammoniaquée par une solution d'acécate de cuivre diammine dans l'ammoniaque. On laisse l'échange se faire pendant 4 heures, on sèche, on réduit à 280°C sous un courant d'azote contenant 3 % d'hydrogène. La mesure du cuivre de surface par la méthode à l'oxyde d'azote donne 1,59 $10^{-3}$ atome-gramme de cuivre par gramme de catalyseur. On dépose sur le précurseur réduit 0,30 $10^{-3}$ atome-gramme de ruthénium par gramme de précurseur à partir d'une solution aqueuse de chlorure de ruthénium que l'on laisse en contact 4 heures avec le solide. On sèche sous vide le catalyseur qui constitue le catalyseur C des exemples suivants.

Exemple 2 :

1°) Préparation d'un précurseur au cuivre

Dans un ballon équipé d'une calotte chauffante, d'un système de réfrigération interne ainsi que d'un agitateur, on introduit un litre d'une solution à 40 % d'hydroxyde de sodium. A travers un système de sas permettant d'introduire un solide en isolant l'intérieur du ballon de l'air extérieur, on laisse tomber progressivement, par fractions de 2 grammes toutes les 2 minutes, 20 grammes d'alliage de Raney (50 % Cu - 50 % Al) en poudre fine. On maintient ensuite la température à 60°C pendant 3 heures. On lave alors le solide à l'eau. Le solide obtenu renferme 99 % de cuivre et présente 0,49 $10^{-3}$ atome-gramme de cuivre en surface par gramme. Ce solide constitue le catalyseur D (précurseur) .

2°) On reprend la préparation précédente jusqu'à obtention du catalyseur D. On introduit alors une solution contenant 13 mg de ruthénium/gramme de précurseur (soit 0,128 $10^{-3}$ atome-gramme de ruthénium) sous la forme d'une solution de chlorure que l'on laisse 5 heures au contact du solide précédent. On lave à l'eau et on sèche sous vide. Le catalyseur obtenu est le catalyseur E.

3°) On reprend la préparation ci-dessus, mais au lieu d'introduire une solution de chlorure de ruthénium, on introduit une solution d'acide chloroplatinique contenant 25 mg de platine/gramme de précurseur (soit 0,128 $10^{-3}$ atome-gramme de platine). Le catalyseur obtenu est le catalyseur F.

4°) On reprend la préparation ci-dessus mais au lieu d'introduire une solution de chlorure de ruthénium, on introduit une solution de chlorure de palladium à raison de 15 mg de palladium par gramme de précurseur (soit 0,14 $10^{-3}$ atome-gramme de palladium). Le catalyseur obtenu est le catalyseur G.

5°) On reprend la préparation du paragraphe 1 jusqu'à l'obtention du cuivre de Raney mais au lieu d'introduire du chlorure de ruthénium, on introduit une solution d'acide chloroaurique à raison de 48 mg d'or par gramme de précurseur (soit 0,24 $10^{-3}$ atome-gramme d'Or). Le catalyseur obtenu est le catalyseur H.

Exemple 3 :

1°) Préparation d'un précurseur

On mélange 59,7g de carbonate basique de cuivre et 55g de ciment Sécar (marque de commerce de la Société des Ciments Lafarge). Ce ciment a la composition moyenne suivante (en % poids) $Al_2O_3$ = 71 ; CaO = 27 ; $Na_2O$ = 0,35 ; $SiO_2$ = 0,35 ; $Fe_2O_3$ = 0,25 et contient moins de 0,2 % poids de chacun des autres constituants. La poudre ainsi obtenue est broyée afin d'atteindre une granulométrie inférieure à 2 micromètres et mise en forme en billes comme il est enseigné par exemple dans le brevet US 4,257,920.

Le solide obtenu a un volume poreux total de 30 ml/100g et une surface spécifique de 149 m2/g. La surface de cuivre mesurée par titrage avec $N_2O$ indique 0,35 $10^{-3}$ atome-gramme de cuivre par gramme de catalyseur, le catalyseur obtenu constitue le catalyseur I (précurseur).

2°) On réduit le solide obtenu par traitement à 280°C pendant 2 heures sous courant d'hydrogène dilué à 3 % dans de l'azote, puis on l'introduit, à l'abri de l'air, dans un ballon contenant une solution de chlorure de ruthénium à raison de 6 mg de ruthénium par gramme de solide (soit 0,06 $10^{-3}$ atome-gramme de ruthénium). Le solide obtenu, après séchage, constitue le catalyseur J.

3°) On reprend la même fabrication du précurseur au cuivre que ci-dessus jusqu'à la mise en forme sous forme de billes, mais l'on dépose le ruthénium en quantité équivalente sous forme de ruthénium pentacarbonyle en solution dans l'heptane. Le solide est filtré, séché sous vide et constitue le catalyseur K.

Exemple 4 (comparatif)

1°) On dépose 4 % en poids de palladium sur du charbon poudre d'une surface de 1100 m2/g à partir de chlorure de palladium en solution dans l'eau que l'on précipite par ajout de soude. Le catalyseur préparé, une fois séché, constitue le catalyseur L.

2°) On dépose de la même manière que ci-dessus 3 % en poids de ruthénium sur charbon à partir d'une solution aqueuse de chlorure de ruthénium. Le catalyseur préparé, une fois séché, constitue le catalyseur M.

Les exemples suivants décrivent les transformations que l'on peut réaliser avec ces catalyseurs. Dans tous les cas la caractérisation a été effectuée par couplage chromatographie-spectrométrie de masse pour la séparation et l'identification des produits après les avoir peracétylés par l'anhydride acétique, ainsi que par la RMN (Résonance magnétique nucléaire) du carbone 13.

Exemple 5 (comparatif)

Dans un réacteur en verre équipé d'un système d'agitation, on introduit 500 cm3 d'une solution sulfurique de sorbitol contenant 150 g de sorbitol, la normalité de la solution sulfurique étant de 2,5. On porte l'ensemble sous reflux. Après 7 heures ou 200 heures de réaction, on refroidit l'ensemble et on analyse le mélange obtenu. Dans le fond du ballon, on recueille un produit très visqueux que l'on appelle "polymères" dans le tableau I, dans lequel les résultats obtenus sont indiqués en grammes, ainsi qu'en pourcentages par rapport au poids total du mélange.

Tableau I

| Durée de la réaction | 7 heures | 200 heures |
|---|---|---|
| Sorbitol | 73,5 g (51,2 %) | traces ($\leqslant$0,2 %) |
| Mélange de divers monoanhydro-sorbitols | 54,6 g (38,0 %) | 42 g (32,5 %) |
| 1,4-3,6 dianhydrosorbitol | 1,7 g (1,2 %) | 65 g (50,3 %) |
| Polymères | 13,8 g (9,6 %) | 22 g (17,0 %) |

Exemple 6 : ( selon l'invention)

Les catalyseurs en poudre précédemment dénommés A,B, E, F, G, H sont testés de la façon suivante :

On réduit 5 grammes de catalyseur à 280°C sous un courant de 10 l/h d'azote contenant 3% volume d'hydrogène. Sans remettre à l'air le catalyseur réduit, on l'introduit, sous gaz inerte, dans 200 cm3 d'une solution aqueuse de sorbitol à raison de 300 g/litre contenue dans un réacteur agité de type Grignard de 500 ml. On effectue la réaction dans les conditions suivantes :

Agitation : 1200 tours/minute

Pression d'hydrogène : 4.10⁶ Pa

Température : 260°C

On prélève un échantillon de solution pour analyses au bout de cinq heures de réaction. Les résultats sont présentés dans le Tableau II et sont exprimés en grammes de produits contenus dans le réacteur, ainsi qu'en pourcentages par rapport au poids total du mélange.

Tableau II

| CATALYSEUR | A | B | E | F | G | H |
|---|---|---|---|---|---|---|
| SORBITOL | traces ($\leq 0,2\%$) | traces ($\leq 0,2\%$) | traces ($\leq 0,2\%$) | traces ($\leq 0,2\%$) | traces ($\leq 0,2\%$) | traces ($\leq 0,2\%$) |
| COMPOSES MONO-ANHYDROSORBITOLS | 12,3 (23,1%) | 30,1 (57,1%) | 10,7 (20,8%) | 21,5 (40,1%) | 22,1 (40,5%) | 9,2 (17,5%) |
| COMPOSES DIANHY-DROSORBITOLS | 21,9 (41,1%) | 15,7 (29,8%) | 30,1 (58,4%) | 16,4 (30,6%) | 15,9 (29,2%) | 26,8 (51,0%) |
| POLYOLS LINEAIRES | 15,2 (28,5%) | 4,7 (8,9%) | 8,5 (16,5%) | 12,5 (23,3%) | 13,2 (24,2%) | 13,1 (25,0%) |
| POLYMERES | 3,8 (7,1%) | 2,1 (4,0%) | 2,1 (4,1%) | 3,1 (5,8%) | 3,2 (5,9%) | 3,3 (6,3%) |

On voit donc que la réaction effectuée sur des catalyseurs métalliques fournit des quantités importantes de produits anhydrosorbitols et que la coproduction de polymères est limitée.

Exemple 7

On effectue la même manipulation que dans l'exemple 6 mais on remplace la solution de sorbitol par une solution de mannitol. Les résultats obtenus après cinq heures de réaction avec les catalyseurs A et H sont présentés en grammes de produits formés dans le Tableau III, ainsi qu'en pourcentages par rapport au poids total de mélange.

## Tableau III

| CATALYSEUR | A | H |
|---|---|---|
| MANNITOL | traces ($\leqslant$ 0,2%) | traces ($\leqslant$ 0,2 %) |
| COMPOSES MONOANHYDROMANNITOLS | 12,0 (22,6%) | 9,0 (17,2%) |
| COMPOSES DIANHYDROMANNITOLS | 22 (41,5%) | 26,7 (51,0%) |
| POLYOLS LINEAIRES | 14,8 (27,9%) | 13,6 (26,9%) |
| POLYMERES | 4,1 (7,8%) | 3,0 (5,7%) |

<u>Exemple 8 (Comparatif)</u>

Les catalyseurs dénommés D, L, M sont testés dans les mêmes conditions que dans l'exemple 6; les résultats obtenus après cinq heures de réaction sont présentés dans le Tableau IV. Les quantités sont données en grammes.

Tableau IV

| CATALYSEUR | D | L | M |
|---|---|---|---|
| SORBITOL | traces | traces | traces |
| COMPOSES MONOANHYDRO-SORBITOLS | 1,2 | non détecté | non détecté |
| COMPOSES DIANHDROSORBITOLS | 0,5 | non détecté | non détecté |
| POLYOLS LINEAIRES | 29 | traces | traces |
| POLYMERES | 3 | 1,2 | 1,4 |

Le catalyseur D a une mauvaise conversion et donne principalement des polyols linéaires.

Les catalyseurs L et M dégradent profondément la molécule, produisant de grandes quantités de gaz carbonique et de méthane.

Exemple 9

Les catalyseurs sous forme de billes et d'extrudés précédemment dénommés C, I, J, K sont testés de la façon suivante :

On charge 20 cm3 de catalyseur dans un réacteur tubulaire et on le traite pendant 2 heures à 280°C par un courant d'azote contenant 3% d'hydrogène.

Après refroidissement, on introduit en continu une solution aqueuse de sorbitol à 250 g/l. Les conditions opératoires de la réaction sont les suivantes :

Pression : $4.10^6$ Pa

Température : 260°C

Débit d'hydrogène : 15 l/h

Débit solution de sorbitol : 20 cm3/h

Les produits obtenus sont analysés comme précédemment. Les tableaux V et VI présentent les résultats obtenus respectivement après 5 heures et après 500 heures de fonctionnement du catalyseur . Les chiffres indiqués sont exprimés en g/l et en pourcentage par rapport à la concentration totale.

Tableau V

Produits formés après 5 h de fonctionnement.

| CATALYSEURS | C | J | K | I* |
|---|---|---|---|---|
| SORBITOL | 5,1g/l (2,6%) | 15,3g/l (7,0%) | 17,5g/l (8,2%) | 17,2g/l (10,3%) |
| MONOANHYDRO-SORBITOLS | 45,2g/l (23,0%) | 50,5g/l (23,2%) | 43,2g/l (20,2%) | non détecté |
| DIANHYDRO-SORBITOLS | 110,5g/l (56,1%) | 90,5g/l (41,3%) | 92,7g/l (43,2%) | non détecté |
| POLYOLS LINEAIRES | 36,0g/l (18,3%) | 62,7g/l (28,6%) | 60,8g/l (28,4%) | 15Gg/l (89,7%) |

* Comparaison : le catalyseur au cuivre seul (précurseur) dégrade de façon importante la molécule de sorbitol et favorise la formation de polyols linéaires.

Tableau VI

Produits formés après 500 h de fonctionnement

| CATALYSEURS | C | J | K | I* |
|---|---|---|---|---|
| SORBITOL | 14,8g/l (8,6%) | 16,2g/l (7,6%) | 18,0g/l (8,6%) | 19,5g/l (11,9%) |
| MONOANHYDRO-SORBITOLS | 42,5g/l (24,6%) | 47,8g/l (22,5%) | 42,7g/l (20,4%) | non détecté |
| DIANHYDRO-SORBITOLS | 80,2g/l (46,4%) | 85,8g/l (40,3%) | 90,5g/l (43,3%) | non détecté |
| POLYOLS LINEAIRES | 35,2g/l (20,4%) | 63,0g/l (29,6%) | 58,0g/l (27,7%) | 144,0 g/l (88,1%) |

\* Comparaison : le catalyseur I dégrade de façon importante la molécule de sorbitol et favorise la formation de polyols linéaires.

Exemple 10

Le catalyseur J a été testé dans les mêmes conditions que dans l'exemple 9 mais la solution contenant 250 g de mannitol par litre.

Les résultats obtenus après 5 heures et 500 heures de fonctionnement sont présentés dans le tableau VII.

EP 0 380 402 B1

Tableau VII

Catalyseur J

| FONCTIONNEMENT | 5 heures | 500 heures |
|---|---|---|
| MANNITOL | 16,4g/1 (7,6 %) | 18,1g/1 (8,6 %) |
| MONOANHYDROMANNITOL | 48,2g/1 (22,3 %) | 45,4g/1 (21,5 %) |
| DIANHYDROMANNITOL | 91,6g/1 (42,3 %) | 86,2g/1 (40,9 %) |
| POLYOLS LINEAIRES | 60,1g/1 (27,8 %) | 61,0g/1 (29,0%) |

## Revendications

1. Procédé de production d'au moins un composé choisi parmi les anhydrosorbitols et les anhydromannitols, dans lequel on fait réagir de l'hydrogène avec au moins un composé choisi parmi le sorbitol et le mannitol, en présence d'un catalyseur renfermant du cuivre, caractérisé en ce que le catalyseur renferme en outre au moins un second métal choisi parmi les métaux nobles du groupe VIII et l'or.

2. Procédé selon la revendication 1, dans lequel le métal noble du groupe VIII est choisi dans le groupe formé par le palladium, le platine, et le ruthénium.

3. Procédé selon la revendication 1 ou 2, dans lequel on utilise un catalyseur renfermant 10-99% en poids de cuivre et 0,01-10% en poids du second métal.

4. Procédé selon l'une des revendications 1 à 3, dans lequel on utilise de 0,1 à 0,8 atome-gramme de second métal par atome-gramme de cuivre en surface, déterminé par titrage au protoxyde d'azote.

5. Procédé selon l'une des revendications 1 à 4, dans lequel on opère à 200-280°C sous $10^5$ à $10^7$ Pa de pression d'hydrogène.

6. Procédé selon la revendication 5, dans lequel on opère à 220 - 260°C sous $10^6$ à $5.10^6$ Pa de pression d'hydrogène.

## Patentansprüche

1. Verfahren zur Herstellung von mindestens einer Anhydrosorbit- oder Anhydromannit-Verbindung, bei dem man Wasserstoff mit mindestens einer Sorbit- oder Mannit-Verbindung in Anwesenheit eines Katalysators, der Kupfer enthält reagieren läßt, dadurch gekennzeichnet, daß der Katalysator außerdem mindestens ein zweites Metall enthält, das unter den Edelmetallen der Gruppe VIII und Gold gewählt ist.

2. Verfahren nach Anspruch 1, bei dem als Edelmetall der Gruppe VIII Palladium, Platin oder Ruthenium

11

gewählt wird.

3. Verfahren nach Anspruch 1 oder 2, bei dem ein Katalysator verwendet wird, der 10 bis 99 Gew.-% an Kupfer und 0,01 bis 10 Gew.-% des zweiten Metalls enthält.

4. Verfahren nach einem der Ansprüche 1 bis 3, bei dem zwischen 0,1 und 0,8 Grammatome des zweiten Metalls pro Grammatome des Kupfers an der Oberfläche verwendet werden das durch Stickstoffmonoxidtitration bestimmt wird.

5. Verfahren nach einem der Ansprüche 1 bis 4, das bei 200 bis 280°C und unter einem Wasserstoffdruck von $10^5$ bis $10^7$ Pa durchgeführt wird.

6. Verfahren nach Anspruch 5, das bei 220 bis 260°C unter einem Wasserstoffdruck von $10^6$ bis $5 \times 10^6$ Pa durchgeführt wird.


**Claims**

1. Process for the production of at least one compound chosen from among anhydrosorbitols and anhydromannitols, in which the hydrogen is reacted with at least one compound chosen from among sorbitol and mannitol, in the presence of a catalyst containing copper, characterized in that the catalyst also contains at least one second metal chosen from among the noble metals of group VIII and gold.

2. Process according to claim 1, wherein the noble metal of group VIII is chosen from among palladium, platinum and ruthenium.

3. Process according to claims 1 or 2, wherein use is made of a catalyst containing 10 to 99% by weight copper and 0.01 to 10% by weight of the second metal.

4. Process according to any one of the claims 1 to 3, wherein use is made of 0.1 to 0.8 gram atoms of the second metal per gram atom of surface copper, determined by nitrous oxide titration.

5. Process according to any one of the claims 1 to 4, wherein working takes place at 200 to 280°C under $10^5$ to $10^7$ Pa hydrogen pressure.

6. Process according to claim 5, wherein working takes place at 220 to 260°C under $10^6$ to $5.10^6$ Pa hydrogen pressure.